# NEUE EUROPÄISCHE PATENTSCHRIFT

(11) **EP 1 301 583 B2**
(45) Veröffentlichungstag und Bekanntmachung des Hinweises auf die Entscheidung über den Einspruch: **11.07.2012**
(45) Hinweis auf die Patenterteilung: 06.10.2004
(21) Anmeldenummer: 01955353.6
(22) Anmeldetag: 16.07.2001
(51) Int. Cl.: C12M 1/107

(54) **BIOREAKTOR ZUR METHANISIERUNG VON BIOMASSE UND EINE BIOGASANLAGE ZUR ERZEUGUNG VON THERMISCHER, ELEKTRISCHER ODER MECHANISCHER ENERGIE AUS BIOMASSE MIT EINEM SOLCHEN BIOREAKTOR SOWIE VERFAHREN ZUR REGELUNG UND STEUERUNG EINER SOLCHEN BIOGASANLAGE**
BIOREACTOR FOR METHANISING BIOMASS AND A BIOGAS INSTALLATION FOR PRODUCING THERMAL, ELECTRIC OR MECHANICAL ENERGY FROM BIOMASS USING SAID BIOREACTOR, METHOD FOR REGULATING AND CONTROLLING ONE SUCH BIOGAS INSTALLATION
BIOREACTEUR DESTINE A LA METHANISATION DE BIOMASSE, DISPOSITIF DE GAZ BIOLOGIQUE DESTINE A LA PRODUCTION D'ENERGIE THERMIQUE, ELECTRIQUE OU MECANIQUE A PARTIR DE BIOMASSE, COMPORTANT UN TEL BIOREACTEUR, ET PROCEDE DE REGULATION ET COMMANDE D'UN TEL DISPOSITIF DE GAZ BIOLOGIQUE

(30) Priorität: 14.07.2000 DE 10034279; 25.09.2000 DE 10047373; 08.03.2001 DE 20104047 U
(43) Veröffentlichungstag der Anmeldung: 16.04.2003
(73) Patentinhaber: Bekon Energy Technologies GmbH & CO. KG, 85774 Unterföhring (DE)
(72) Erfinder: LUTZ, Peter, 85413 Hörgertshausen (DE)
(74) Vertreter: Winter, Brandl, Fürniss, Hübner, Röss, Kaiser, Polte - Partnerschaft
(86) Internationale Anmeldenummer: PCT/EP2001/008200
(87) Internationale Veröffentlichungsnummer: WO 2002/006439

(56) Entgegenhaltungen:
- EP-A- 0 023 176
- EP-A- 0 048 675
- EP-A- 0 142 473
- EP-A- 0 822 251
- DE-A- 19 803 389
- FR-A- 2 502 174

## Beschreibung

Die Erfindung betrifft einen Bioreaktor zur Methanisierung von Biomasse, eine mit einem solchen Bioreaktor arbeitende Biogasanlage zur Erzeugung von thermischer, elektrischer oder mechanischer Energie aus Biomasse und ein Verfahren zur Steuerung und Regelung der Biogasanlage.

Zur Erzeugung von Biogas aus Biomasse aus der Landwirtschaft oder aus Bioabfällen ist aus der EP 0934 998 ein sogenanntes Trockenfermontations-Verfahren zur Methanisierung von halbfeuchter, schüttfähiger, stapelbarer oder stückigmachbarer, inokulierter Biomasse bekannt. Der in einem gasdichten Kontainer eingelagerten Biomasse wird ein impfmaterial zugesetzt und die so gebildete Reakionsmasse wird unter Luftabschluß vergoren.

Aus der EP 0 755 905 A1 ist ein Bioreaktor zur Methanisierung von Biomasse bekannt, bei dem die Biomasse in eine Art Fahrsilo gefüllt und mit einer Plane gasdicht abgedeckt wird. Auf dem Boden und in den Seitenwänden des Fahrsilos sind nuten vorgesehen in denen Heizungsrohre angeordnet sind. Um die Heizungsrohre vor Beschädigung zu schützen, werden sie durch Halbschalen aus Eisen abgedeckt.

Es ist Aufgabe der vorliegenden Erfindung, für dieses Trockenfermentationsverfahren einen kostengünstigen Bioreaktor anzugeben. Weiter ist es Aufgabe der Erfindung eine sichere Biogesanlage anzugeben. Weiter ist es Aufgabe der vorliegenden Erfindung erfahren zum Betreiben und zur Steuerung derartiger Biogasanlagen anzugeben.

Die Lösung dieser Aufgaben erfolgt durch die Merkmale der Ansprüche 1, 8, 17, 19 bzw. 20.

Der Bioreaktor nach Anspruch 1 weist einen sehr einfachen Aufbau auf. Durch die gasdicht verschließbare Klappe, die ausreichend grob ausgeführt ist, kann auf einfache Weise Biomasse In den Behälter eingefüllt und die Biorestmasse kann nach der Methanisierung leicht wieder entnommen werden. Durch die flächige in der Behälterwandung vorgesehene Heizvorrichtung wird die für die Methanisierung notwendige Temperatur bereitgestellt. Darüber hinaus kann durch die Regelung der Heizung auf den Vergasungsprozeß eingewirkt werden. Der vorzugsweise über die Sickersaft-Drainageeinrichtung abgeführte Sickersaft kann, gegebenenfalls nach Aufbereitung wieder dem Faulbehälter zugeführt werden. Der Faulbehälter ist nach Art einer Fertiggarage aus Stahlbeton aufgebaut. Die offene Seite der "Fertiggarage" wird durch die Klappe gasdicht verschlossen. Hierdurch ergibt sich eine sehr kostengünstige Konstruktion. Die Heizeinrichtung ist nach Art einer Fußbodenheizung in die Bodenplatte Faulbahäfters integriert. Da warme Gase nach oben steigen, wird hierdurch eine gleichmäßige Durchwärmung der Biomasse In dem Faulbehälter erreicht. Zusätzlich oder alternativ läßt sich die Heizeinrichtung auch in die übrige Behälterwandung integrieren.

Gemäß einer vorteilhaften Ausgestaltung der Erfindung nach Anspruch 2 und 3 ist die den Faulbehälter gasdicht abschließende Klappe mit einem aufblasbaren Dlchtungsschlauch versehen. In geschlossenem Zustand wird der Dichtungsachlauch aufgeblassen und dichtet die Klappe gegenüber der Behälterwandung auf einfache Weise gasdicht ab.

Gemäß einer weiteren vorteilhaften Ausgestaltung der Erfindung nach Anspruch 4 ist die Klappe hydraulisch betätigbar, da diese bei entsprechenden Dimensionen kaum mehr von Hand betätigbar ist.

Gemäß einer vorteilhaften Ausgestaltung der Erfindung nach Anspruch 5 ist der Faulbehälter kubisch oder quaderförmig ausgeführt, wobei die Klappe eine Wandung des Kubus oder Quaders bildet. Hierdurch ergibt sich zum einen eine einfache Konstruktion und zum anderen eine ausreichend große Öffnung zum Beladen und Befüllen des Faulbehälters. Zusätzlich vereinfacht sich dadurch die Herstellung des Faulbehälters.

Gemäß einer vorteilhaften Ausgestaltung der Erfindung nach Anspruch 6 ist die Decke des Faulbehälter mittels Hubzylindern anhebbar und wieder gasdicht verschließbar. Hierdurch wird eine schnelle Belüftung des Faulbehälter gewährleistet.

Gemäß einer vorteilhaften Ausgestaltung der Erfindung nach Anspruch 7 ist der Faulbehälter zylindrisch ausgebildet und die Klappe besitzt die Form eines scheibenförmigen Deckels. Diese Form ist insbesondere für Rundstrohballen als Biomasse geeignet.

Gemäß Anspruch 8 wird eine Biogasanlage mit einem erfindungsgemäßen Bioreaktor bereitgestellt.

Gemäß Anspruch 9 wird eine Biogasanlage, insbesondere mit einem erfindungsgemäßen Bioreaktor bereitgestellt, der die Zwischeniagerung von in dem Bioreaktor erzeugten Biogas unnötig macht. Dies geschieht durch eine Regelung gemäß Anspruch 17. Dadurch, dass der Biogasverbraucher, z.B. ein Biockheizkraftwerk, ein Verbrennungskessel, eine Brennstoffzeile, usw., in unterschiedlichen Lastbereichen gefahren wird, wird erreicht, dass jeweils nur das erzeugte Biogas verbraucht wird. Folglich erübrigt sich eine Zwischenspeicherung des erzeugten Biogases. Die Zufuhr von Biogas aus den Biogasreaktoren zu dem Biogasverbraucher wird über eine Differenzdruckerfassungseinrichtung und eine Biogasverbraucherregeteinrichtung so gesteuert, daß die Druckdifferenz in dem jeweiligen Biogasreaktor und dem Umgebungsdruck Innerhalb eines bestimmten Druckintervalls liegt. Steigt der Differenzdruck zwischen Gasdruck im Bioreaktor und Umgebungsdruck an, wird mehr Biogas der Biogasverbrauchereinrichtung zugeführt, so daß diese mit höherer Leistung thermische, elektrische oder mechanische Energie erzeugt. Sinkt der Differenzdruck ab, wird die Zufuhr von Biogas zu der Biogasverbrauchereinrichtung gedrosselt und damit die Leistung der Biogasverbrauchereinrichtung heruntergefahren.

Auf diese Weise erübrigen sich Behälter z.B. In Ballonform, zum Zwischenlagern des in den Bioreaktoren erzeugten Biogases. Ein erhebliches Gefahrenpotential solcher Biogaszwischenlagerbehälter ist damit völlig ausgeschaltet und die Sicherheit von Biogasanlagen wird erheblich erhöht.

Die Biagasanlage gemäß Anspruch 11 zeichnet sich ebenfalls durch eine erhöhte Sicherheit aus. Regelungstechnisch wird dies durch ein Verfahren nach Anspruch 19 erreicht. Wenn Biogasreaktoren undicht werden, kann sich in dem Bioreaktor ein leicht entzündliches, explosives Biogas/Sauerstoff-Gemisch bilden. Aufgrund von Funkenentladung, Zigaretten oder statischer Elektrizität kann es damit zu schweren Explosionen kommen. Bei der Biogasanlage gemäß Anspruch 11 wird der Sauerstoffpartialdruck in dem jeweiligen Bioreaktor gemessen bzw. kontinulerlich überwacht. Übersteigt der Sauerstoffpartialdruck einen bestimmten Wert In dem jeweiligen Bioreaktor, ist dies ein Anzeichen dafür, daß ein Leck aufgetreten ist und Sauerstoff eindringt. Um diesen gefährlichen Zustand zu verhindem, wird bei Überschreiten eines Schwellwertes für den Sauerstoffpartialdruckes der jeweilige Bioreaktor von der Biogasleitung abgesperrt. Gleichzeitig wird Abgas, d.h. im wesentlichen CO₂, aus dem Biogaaverbraucher über über eine Abgasspülleitung in den Bioreaktor mit dem erhöhten Sauerstoffpartialdruck geführt und ein Spülventil in dem Bioreaktor geöffnet, so daß die in den Bioreaktor befindlichen Gase aus dem Bioreaktor entwelchen können und schließlich nahezu ausschließlich Kohlendioxid In dem Bioreaktor verbleibt. Ist der jeweilige vermutlich lecke Bioreaktor mit Kohlendioxid bzw. Abgas geflutet, kann er ohne Explosionsgefahr geöffnet werden und anschließend repariert werden.

Die Biogasanlage gemäß Anspruch 13 zeichnet sich durch eine hohe Betriebssicherheit aus. Dies wird dadurch erreicht, daß dem aus dem jeweiligen Bioreaktor austretenden Sickersaft oder Perkulat entsprechend der jeweiligen Zusammensetzung Zusafzstoffe hinzugefügt werden, bevor diese in dem Bioreaktor zurückgeführt werden. Hierdurch ist es möglich, die Vergasungsreaktion im Bioreaktor positiv zu beeinflussen. Beispielsweise wird der ph-Wert des Perkulats bzw. des austretenden Sickersafts vor der Rückführung erfaßt und falls der ph-Wert zu niedrig ist, kann Lauge, insbesondere Kalziumhydroxid oder Kalkmilch In entsprechenden Mengen zugesetzt werden (Anspruch 14). Darüber hinaus lassen sich durch Messung signifikanter Parameter, wie Zusammensetzung, Feststoffgehalt etc. des Sickersaftes bzw. des Perkulats Rückschlüsse auf den Vergänrungsprozeß im Bioreaktor gewinnen. Durch Zumischung von Zusätzen, z.B. Milchzucker als Nahrung für die am Gärungsprozeß beteiligten Bakterien kann dieser positiv beeinflußt werden und somit die Biogasausbeute erhöht werden (Ansprüche 15 und 19 bis 22).

Die übrigen Unteransprüche beziehen sich auf weitere vorteilhafte Ausgestaltungen der Erfindung.

Weitere Einzelheiten, Merkmale und Vorteile der Erfindung zeigt die nachfolgende Beschreibung bevorzugter Ausführungsformen anhand der Zeichnungen:

Es zeigt:
Fig. 1 schematisch eine perspektivische Darstellung des erfindungsgemäßen Bioreaktors;
Fig. 2 eine Schnittdarsteilung des Bioreaktors nach
Fig. 1;

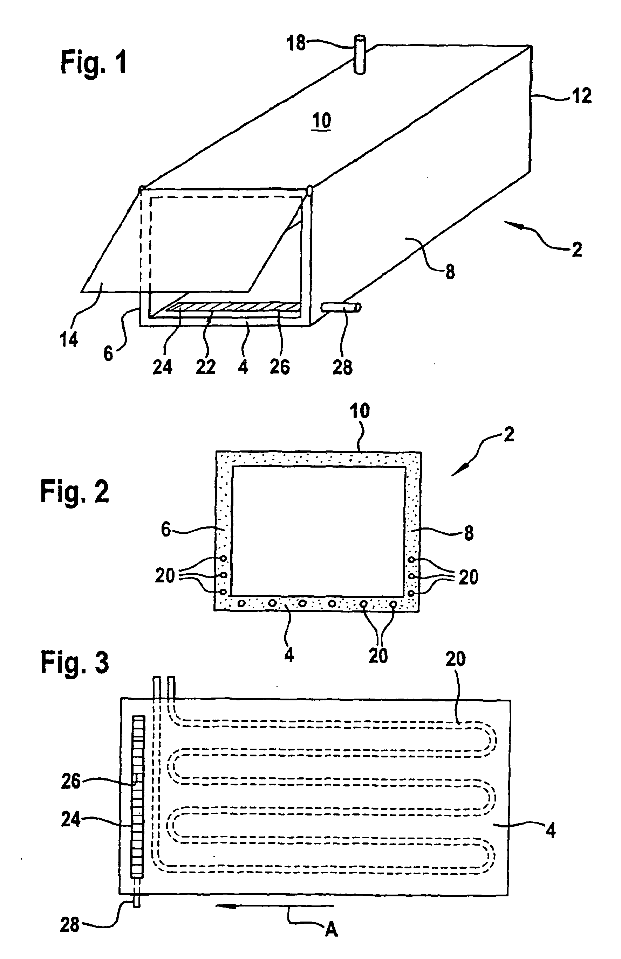

Fig. 3 eine schematische Darstellung der Bodenplatte des Bioreaktors aus Fig. 1 bzw. 2;

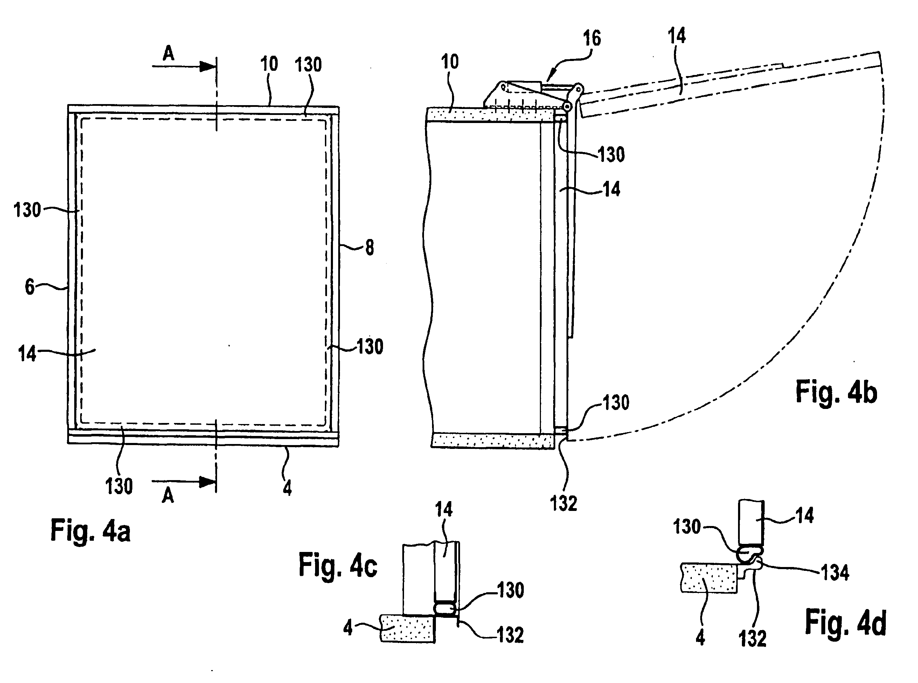

Fig. 4a eine Aufsicht auf die Klappe des Faulbehälters;
Fig. 4b eine Schnittdarsteilung der Klappe aus Fig. 4a entlang der Ebene A-A;
Fig. 4c ein Detail der Darstellung In Fig. 4b;
Fig. 4d eine Fig. 4c entsprechend Detaildarstellung mit einer alternativen Ausgestaltung der Zarge.

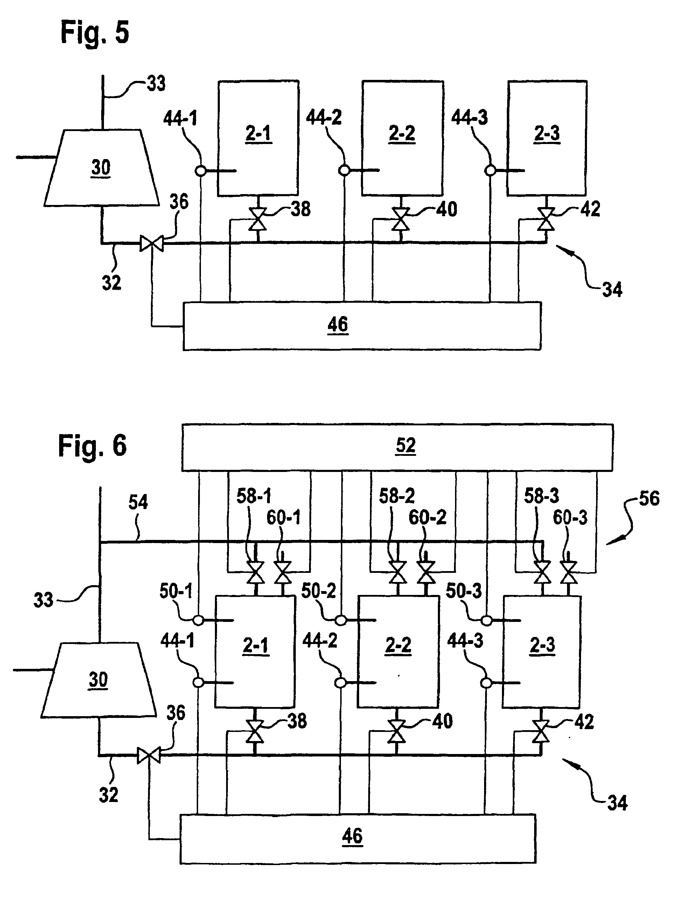

Fig. 6 eine erste Ausführungsform einer Biogasanlage gemäß der vorliegenden Erfindung;
Fig. 6 eine zweite Ausführungsform der Biogasanlage;

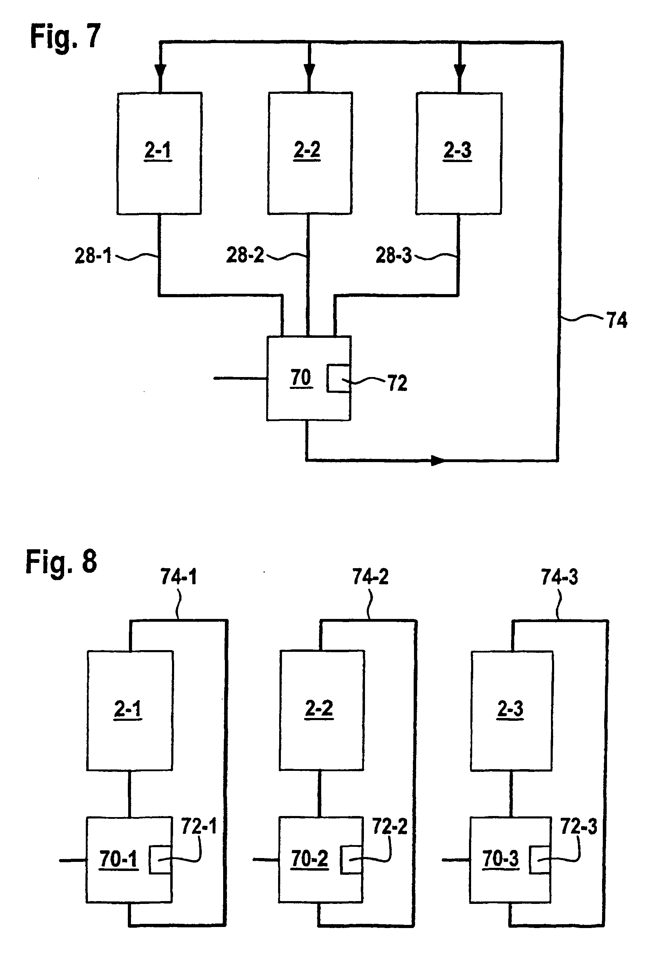

Fig. 7 eine dritte Ausführungsform einer Biogasanlage bzw. eines Bioreaktors;
Fig. 8 eine Variante der Ausführungsform nach Fig. 7;

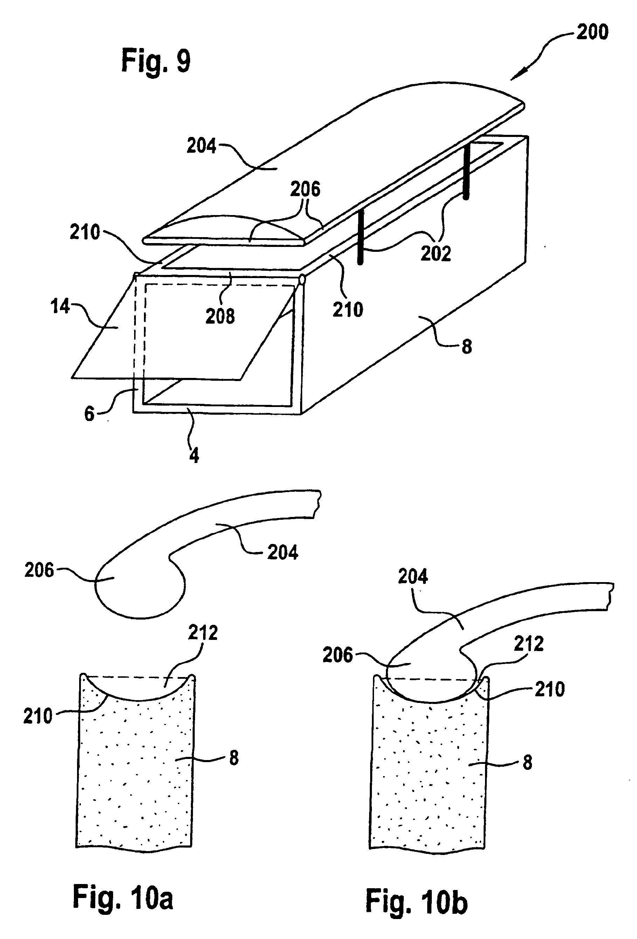

Fig. 9 eine schematische, perspektivische Darstellung einer weiteren Ausführungsform des erfindungsgemäßen Bioreaktors; und
Fig. 10a und 10b Detaildarstellungen der Ausführungform nach Fig. 9.

Der Bioreaktor bzw. Biogasreaktor nach den Figuren 1 bis 3 umfaßt einen quaderförmigen Faulbehälter 2, der nach Art einer Fertiggarage aus Stahlbeton besteht und sechs plane Wandelemente umfaßt, nämlich eine Bodenplatte 4, zwei Seitenwände 6 und 8, eine Deckplatte 10, eine Rückwand 12 und eine offene Vorderseite, die durch eine gasdichte Klappe 14 verschließbar ist.

Die Klappe 14 ist mittels einer Hydraulik 16 betätigbar. Bei offener klappe 14 1äßt sich derFaulbehälter 2 auf einfache Weise befüllen bzw. die Restbiomasse daraus entfernen. Über einen Biogasentnahmeanschluß 18 wird das in dem Faulbehälter 2 erzeugte Biogas abgeführt. In der Bodenplatte 4 des Faulbehälters 2 und teilweise auch in den Seitenwänden 6 und 8 ist eine Heizeinrichtung 20 nach Art einer Fußbodenheizung vorgesehen, mittels der die in dem Faulbehälter 2 befindliche Biomasse entsprechend temperiert werden kann. Ebenfalls In der Bodenplatte 4 integriert ist eine Sickersaft-Drainageeeinrichtung 22, die eine quer verlaufende in die Bodenplatte 4 eingelassene Rinne 24 umfaßt, die durch ein Loch- oder Schlitzblech 26 abgedeckt ist. Über eine Sickersaftableitung 28 wird der sich in der Rinne 24 sammelnde Sickersaft abgeleitet. Die Bodenplatte 4 weist in Richtung des Pfeiles A ein Gefälle hin zur Rinne 24 auf, so daß sich der Sickersaft in der Rinne 24 sammeln kann.

In Fig. 3 ist lediglich eine Rinne 24 dargestellt. Alternativ können mehrere solcher Rinnen vorgesehen werden, die ebenfalls quer oder in Längsrichtung angeordnet sein können.

Fig. 4a zeigt eine Aufsicht auf den Faulbehälter 2 mit geschlossener Klappe 14. Fig. 4b zeigt eine Schnittansicht des Faulbehälters entlang der Ebene A-A in Fig. 4a, wobei strichliert die geöffnete Klappe 14 zusätzlich eingezeichnet ist. Im Randbereich Der Klappe 14 umlaufend ist ein Dichtungschlauch 130 befestigt. Die Klappe 14 greift in geschlossenem Zustand in eine Zarge 132 ein - siehe Fig. 4c -, gegenüber der die Klappe 14 durch Aufpumpen des Dichtungsschlauches 130 auf 6 bar abgedichtet wird.

Fig. 4d zeigt eine alternative Ausgestaltung der Zarge 132, die einen umlaufenden Vorsprung 134 aufweist. Durch den Vorsprung 134 hintergreift der aufgeblasene Dichtungsschlauch 130 die Zarge 132, wodurch die Dichtwirkung noch erhöht wird.

Fig. 5 zeigt eine erste Ausführungsform einer Biogasanlage, bei der vorzugsweise eine Mehrzahl der vorstehend beschriebenen Bioreaktoren zum Einsatz kommen. Die Biogasanlage umfaßt drei Bioreaktoren 2-1, 2-2 und 2-3 sowie einen Biogasverbraucher 30 zum Erzeugen von thermischer, elektrischer und/oder mechanischer Energie aus dem Biogas, z.B. ein Blockheizkraftwerk. Die Bioreaktoren 2-i sind über eine Biogasleitung 32 mit dem Biogasverbraucher 30 verbunden. Über eine Abgasleitung 33 wird Abgas aus dem Biogasverbrraucher abgeführt. Der Zufluß und die Menge von Biogas aus den Bioreaktoren 2-i zum Biogasverbraucher 30 über die Biogasleitung 32 wird mittels einer Ventileinreichtung 34 gesteuert. Die Ventileinrichtung 34 umfaßt ein erstes Ventil 36 in Durchflußrichtung unmittelbar vor dem Biogasverbraucher 30, ein zweites Ventil 38, ein drittes Ventil 40 und ein viertes Ventil 42 jeweils in Durchflußrichtung unmittelbar nach den Bioreaktoren 2-i. Mittels einer Differenzdruckerfassungseinrichtung 44 und einer Biogasverbraucherregeleinrichtung 46 wird die Menge des durch die Biogasleitung 32 zum Biogasverbraucher 30 strömenden Biogases geregelt. Die Differenzdruckerfassungseinrichtung 44 umfaßt drei Differenzdruckmeßeinrichtungen 44-1, 44-2 und 44-3, die jeweils die Differenz zwischen den in den drei Bioreaktoren 2-1, 2-2 und 2-3 herrschenden Gasdruck und dem Umgebungsdruck messen und an die Biogasverbraucherregeleinrichtung 46 weiterleiten. Mittels Computersteuerung wird die über die Biogasleitung 32 in dem Biogasverbraucher 30 strömende Biogasmenge so geregelt, daß der durch die Differenzdruckerfassungseinrichtung 44 erfaßten Differenzdrücke innerhalb eines bestimmten positiven Intervalls bleiben. Dies geschieht durch entsprechende Regelung der Gasflußmenge durch die vier Ventilen 36 bis 42. Der Biogasverbraucher 30 wird also in unterschiedlichen Leistungsbereichen gefahren, je nach dem ob viel Biogas vorliegt oder weniger.

Der große Vorteil hierbei ist, daß eine Zwischenlagerung des in den Bioreaktoren 2-i erzeugten Biogases nicht mehr nötig ist und folglich auch die Explosionsgefahr erheblich verringert ist.

Fig. 6 zeigt eine zweite Ausführungsform der Erfindung, die sich von der Ausführungsform nach Fig. 5 durch eine zusätzliche Sicherheitseinrichtung unterscheidet. Mittels einer Partialdruckmeßeinrichtung 50, die drei den jeweiligen Bioreaktoren 2-1, 2-2, 2-3 zugeordnete Partialdruckmeßstellen 50-1, 50-2 und 50-3 umfaßt, wird der Sauerstoffpartialdruck in den drei Bioreaktoren 2-i ständig überwacht und die Meßwerte werden einer Steuereinrichtung 52 zugeführt. Die Abgasleitung 33 des Biogasverbrauchers 30 ist mit einer Abgasspülleitung 54 verbunden, die in die drei Bioreaktoren 2-i mündet. Mittels einer Ventileinrichtung 56 lassen sich die drei Bioreaktoren 2-i mit Abgas aus dem Biogasverbraucher 30 fluten. Die Ventileinrichtung 56 umfaßt drei den drei Bioreaktoren zugeordnete Ventilpaare mit drei in der Abgasspülleitung 54 angeordneten Absperrventilen 58-1, 58-2 und 58-3 sowie drei Spülventilen 60-1, 60-2 und 60-3, die das Innere der drei Bioreaktoren 2-i mit der Umgebung verbinden.

In normalem Betriebszustand sind die sechs Ventile 58-i und 60-i geschlossen. Mittels der Partialdruckmeßstellen 50-i wird laufend der Sauerstoffpartialdruck in den drei Bioreaktoren 2-i überwacht. Überschreitet der Sauerstoffpartialdruck einen bestimmten Schwellwert, wird davon ausgegangen, daß der Bioreaktor 2-i ein Leck hat und Sauerstoff aus der Umgebung in den Bioreaktor 2-i eindringt und es folglich zu einer explosiven Gemischbildung kommen kann. Um dies zu verhindern, wird bei Überschreiten des Schwellwerts der Bioreaktor mit dem überhöhten Sauerstoffpartialdruck durch Absperren des jeweiligen Ventils 38, 40 oder 42 von der Biogasleitung 32 abgetrennt. Gleichzeitig wird das zugeordnete Absperrventil 58-i und das zugehörige Spülventil 60-i geöffnet und Abgas aus dem Biogasverbraucher 30 in den jeweiligen Bioreaktor 2-i geleitet. Hierdurch wird das in dem Bioreaktor 2-i befindliche Biogas und der eingedrungene Sauerstoff über das Spülventil 60-i an die Umgebung ausgeblasen. Anschließend kann der Bioreaktor 2-i gefahrlos, d.h. ohne Explosionsgefahr, geöffnet und ggfs. repariert werden.

Die anhand von Fig. 6 beschriebene Sicherheitseinrichtung kann auch bei anderen Biogasreaktoren eingesetzt werden.

Fig. 7 und 8 zeigen schematisch eine Einrichtung und ein Verfahren zur Verbesserung der Methanausbeute in Bioreaktoren bzw. Biogasanlagen. In Fig. 7 und 8 sind drei Bioreaktoren 2-i vorgesehen, die entsprechend der Ausführungsform nach Fig. 5 oder 6 mit dem Biogasverbraucher verbunden sind (nicht dargestellt). Die aus den drei Bioreaktoren 2-i abgeführten Sickersäfte werden über drei Sickersaftableitungen 28-1, 28-2 und 28-3 einer Zumischeinrichtung 70 zur Zuführung von Zusatzstoffen zugeführt. Mittels einer Meßeinrichtung 72 werden für die Methanisierung wichtige und signifikante Parameter des Sickersaftes gemessen, z.B. Ph-Wert, Nährstoffgehalt, etc.. Aufgrund der in .der Meßeinrichtung 72 erfaßten Meßwerte werden in der Zumischeinrichtung 70 dem Sickersaft Zusatzstoffe beigefügt, und das Gemisch wird dann über eine Sickersaftrückführleitung als Perkulat in die Bioreaktoren zurückgeführt.

Beispielsweise kann bei Absinken des ph-Werts unter einen bestimmten Wert in der Zumischeinrichtung 70 Kalziumhydroxid oder Kalkmilch zugesetzt werden, so daß der ph-Wert wieder auf einen gewünschten Wert ansteigt. Auch können in der Zumischeinrichtung 70 Nährstoffe und/oder Methanbildner dem Sickersaft zugemischt werden und über die Sickersaftrückführleitung 74 in die Bioreaktoren 2-i eingeführt werden.

Fig. 8 zeigt eine Variante der Ausführungsform nach Fig. 7, wobei jedem Bioreaktor 2-i statt einer gemeinsamen Zumischeinrichtung 70 jeweils eine eigene Zumischeinrichtng 70-i mit zugehöriger Meßstelle 72-i zugeordnet ist. Hierdurch kann die Zumischung zu dem Sickersaft bzw. zum Perkulat individueller auf die Vorgänge in den einzelnen Bioreaktoren 2-i abgestimmt werden.

Fig. 9 zeigt eine zweite Ausführungsform eines Bioreaktors bzw. eines Faulbehälters 200, der sich von der Ausführungsform nach den Figuren 1 bis 4 dadurch unterscheidet, dass anstelle der Deckplatte 10 ein mittels Hubzylindern 202 anhebbarer Deckel 204 vorgesehen ist, der gasdicht auf Seitenwände 6, 8 und Rückwand 12 aufsetzbar ist. Im übrigen entspricht die Ausführungsform nach Fig. 9 der Ausführungsform nach den Figuren 1 bis 4.

Der Deckel 204 ist in seiner Längenerstreckung leicht konvex ausgebildet und weist einen umlaufenden Dichtwulst 206 auf. An der mit der Klappe 14 abschließbaren Stirnseite sind die beiden Seitenwände 6 und 8 an ihrer Oberkante mittels eines Querträger 208 miteinander verbunden. Auf der Oberseite der beiden Seitenwände 6, 8, der Rückwand 12 und dem Querträger 208 ist eine umlaufende Rinne 210 ausgebildet, die teilweise mit einer Flüssigkeit 212 gefüllt ist. In diese Rinne 210 mit Flüssigkeit 212 taucht der Deckel 204 bzw. der umlaufende Dichtwulst 206 ein und verschließt den Faulbehälter 200 gasdicht.

Fig. 10a zeigt eine Schnittdarstellung mit angehobenem Deckel 204 und Fig. 10b zeigt den Deckel 204 in aufgesetztem Zustand, in dem der umlaufende Dichtwulst 206 in die Flüssigkeit 212 in der Rinne 210 eintaucht. Die Hubzylinder 202 können in die Seitenwände 6, 8 integriert oder außen an den Seitenwänden 6, 8 montiert sein. Es werden vorzugsweise Differentialhubzylinder eingesetzt, durch die der Deckel 204 mit dem Dichtwulst 206 in die Rinne 210 eingepreßt wird.

### Bezugszeichenliste:

- 2: Faulbehälter
- 4: Bodenplatte
- 6: Seitenwand
- 8: Seitenwand
- 10: Deckplatte
- 12: Rückwand
- 14: Klappe
- 16: Hydraulik
- 18: Biogasentnahmeanschluß
- 20: Heizeinrichtung, Fußbodenheizung
- 22: Sickersaft-Drainageeinrichtung
- 24: Rinne
- 26: Loch- oder Schlitzblechabdeckung
- 28: Sickersaftableitung

- 130: Dichtungsschlauch
- 132: Zarge
- 134: Vorsprung an Zarge

- 30: Biogasverbraucher
- 32: Biogasleitung
- 33: Abgasleitung
- 34: Ventileinrichtung
- 36: erstes Ventil
- 38: zweites Ventil
- 40: drittes Ventil
- 42: viertes Ventil
- 44-i: Differenzdruckerfassungseinrichtung
- 46: Biogasverbraucherregeleinrichtung
- 50: Partialdruckmeßeinrichtung
- 52: Steuereinrichtung
- 54: Abgasspülleitung
- 56: Ventileinrichtung
- 58-i: Absperrventil
- 60-i: Spülventil
- 28-i: Sickersaftableitung
- 70-i: Zumischeinrichtung
- 72-i: Meßeinrichtung
- 74-i: Sickersaftrückführleitung

- 200: Faulbehälter
- 202: Hubzylinder
- 204: Deckel
- 206: umlaufender Dichtwulst
- 208: Querträger
- 210: umlaufende Rinne
- 212: Flüssigkeit in 210

## Patentansprüche

1. Bioreaktor zur Methanisierung von Biomasse, mit
- einem durch eine Klappe (14) gasdicht verschließbaren Faulbehälter (2; 200) zur Aufnahme der Biomasse;
- einer in der Behälterwandung vorgesehenen flächigen Heizeinrichtung (20);
- einem Biogasentnahmeanschluss (18); und
- einer Sickersaft-Drainageeinrichtung (22);
**dadurch gekennzeichnet, dass**
- der Faulbehälter (2; 200) nach Art einer Fertiggarage aus Stahlbeton ausgebildet ist, und
- dass die flächige Heizeinrichtung (20) nach Art einer Fußbodenheizung in der Bodenplatte (4) des Faulbehälters (2; 200) integriert ist.

2. Bioreaktor nach Anspruch 1, **dadurch gekennzeichnet, dass** die Klappe (14) in ihrem Randbereich mit einem umlaufenden Dichtungsschlauch (130) versehen ist, der mittels einem Fluid, insbesondere Luft, aufblasbar ist.

3. Bioreaktor nach Anspruch 2, **dadurch gekennzeichnet, dass** die Klappe (14) Außenabmessungen aufweist, die kleiner sind als die Innenabmessungen des Faulbehälters (2; 200) in der Ebene in der sich die Klappe (14) in geschlossenem Zustand erstreckt, so dass die Behälterwandung (4, 6, 8, 10; 208) über die Klappe (14) übersteht.

4. Bioreaktor nach einem der vorhergehenden Ansprüche, **dadurch gekennzeichnet, dass** die Klappe (14) mittels einer Hydraulikeinrichtung (16) betätigbar ist.

5. Bioreaktor nach einem der vorhergehenden Ansprüche, **dadurch gekennzeichnet, dass** der Faulbehälter (2; 200) kubisch oder quaderförmig mit vier Seitenwänden (6, 8, 12, 14), Boden (4) und Decke (10; 204) ist und die Klappe (14) eine Wand, vorzugsweise eine senkrecht stehende Seitenwand, des Faulbehälters (2; 200) bildet.

6. Bioreaktor nach Anspruch 5, **dadurch gekennzeichnet, dass** die Decke des Faulbehälter (200) als Deckel (204) ausgebildet ist, der mittels Hubzylindern (202), vorzugsweise Differentialzylindern, anhebbar und absenkbar ist.

7. Bioreaktor nach einem der vorhergehenden Ansprüche 1 bis 4, **dadurch gekennzeichnet, dass** der Faulbehälter die Form eines Zylinder aufweist und die Klappe die Form eines kreisförmigen Deckels aufweist, der eine Stirnseite des Zylinders abdeckt.

8. Biogasanlage zur Erzeugung von thermischer, elektrischer oder mechanischer Energie aus Biomasse, mit
wenigstens einem Bioreaktor (2; 200) zur Erzeugung von Biogas aus Biomasse nach einem der vorhergehenden Ansprüche,
einem Biogasverbraucher (30) zur Erzeugung von thermischer, elektrischer oder mechanischer Energie,
einer Biogasleitung (32) zur Zuführung des Biogases aus dem wenigstens einen Bioreaktor (2, 200) zu dem Biogasverbraucher (30).

9. Biogasanlage nach Anspruch 8, **gekennzeichnet durch**
eine Ventileinrichtung (34) in der Biogasleitung (32) zur Regelung der Biogasdurchflußmenge,
eine Differenzdruckerfassungseinrichtung (44) zur Erfassung des Differenzdrucks zwischen dem in dem wenigstens einen Bioreaktor (2-i) herrschenden Druck und dem Umgebungsdruck, und
eine Biogasverbraucherregeleinrichtung (46) zur Regelung des Verbrauchs von Biogas, derart, dass der in der in der Differenzdruckerfassungseinrichtung (44) erfaßte Differenzdruck in einem bestimmten Regelintervall liegt.

10. Biogasanlage nach Anspruch 9, **dadurch gekennzeichnet, dass** das Regelintervall des Differenzdrucks positiv ist.

11. Biogasanlage nach wenigstens einem der vorhergehenden Ansprüche 8 bis 10, mit
einer Partialdruckmeßeinrichtung (50) zur Erfassung des Sauerstoffpartialdrucks in dem wenigstens einen Bioreaktor (2-i),
einer Abgasspülleitung (54) zur Zuführung von Abgasen aus dem Biogasverbraucher (34) in den wenigstens einen Bioreaktor (2-i),
einer Ventileinrichtung (58-i, 60-i) für jeden Bioreaktor (2-i) zum Verbinden der Abgasspülleitung (54) mit dem jeweiligen Bioreaktor (2-i) und zum Verbinden des jeweiligen Bioreaktors (2-i) mit der Umgebung, und
einer Steuereinrichtung (52) zum Betätigen der Ventileinrichtung (58-i, 60-i) und zum Fluten des jeweiligen Bioreaktors (2-i), wenn der Sauerstoffpartialdruck in dem jeweiligen Bioreaktor (2-i) einen bestimmten Wert überschreitet.

12. Biogasanlage nach Anspruch 11, **dadurch gekennzeichnet, dass** die Ventileinrichtung (58-i, 60-i) für jeden Bioreaktor (2-i) ein Ventilpaar bestehend aus Absperrventil (58-i) in der Abgasspülleitung (54) und Spülventil (60-i) zur Umgebung umfaßt.

13. Biogasanlage nach einem der vorhergehenden Ansprüche 8 bis 12, mit
einer Sickersaft-Drainageeinrichtung (22),
einer Sickersaftrückführleitung (28, 74) zur Rückführung des durch die Sickersaft-Drainageeinrichtung (22) gesammelten Sickersafts in den wenigstens einen Bioreaktor (2-i),
einer Meßeinrichtung (72) zum Erfassen von signifikanten Parametern des durch die Sickersaft-Drainageeinrichtung (22) gesammelten Sickersaftes, und
einer Zumischeinrichtung (70) zur Zuführung von Zusatzstoffen in die Sickersaftrückführleitung (74) entsprechend den durch die Meßeinrichtung (72) erfaßten Parametern.

14. Biogasanlage nach Anspruch 13, **dadurch gekennzeichnet, dass** die Sickersaftmeßeinrichtung (72) den ph-Wert des Sickersaftes mißt und dass die Zumischeinrichtung (70) zur Zuführung von Lauge, insbesondere von Kalziumhydroxid oder Kalkmilch ausgelegt ist, wenn der ph-Wert des Sickersaftes unter einen bestimmten Wert sinkt.

15. Biogasanlage nach Anspruch 13 oder 14, **dadurch gekennzeichnet, dass** die Zumischeinrichtung (70) zur Zuführung von Nährstoffen, insbesondere von Milchzucker, für die in den Bioreaktoren (2-i) aktiven Bakterien ausgelegt ist.

16. Biogasanlage nach einem der vorhergehenden Ansprüche 8 bis 15, **dadurch gekennzeichnet**, das mehrere Biogasverbraucher (30) vorgesehen sind.

17. Verfahren zur Regelung einer Biogasanlage nach einem der Ansprüche 8 bis 16 mit folgenden Verfahrensschritten:
a) Überwachen des Differenzdrucks zwischen dem Druck im Inneren des jeweiligen Bioreaktors (2-i) und der Umgebung, und
b) Regeln der von dem Biogasverbraucher (30) verbrauchten Biogasmenge derart, dass der bzw. die überwachten Differenzdrücke in einem vorbestimmten Druckintervall verbleiben.

18. Verfahren nach Anspruch 17, **dadurch gekennzeichnet, dass** die Regelung der von dem Biogasverbraucher (30) verbrauchten Biogasmenge durch Variation der Biogasdurchflußmenge in der Biogasleitung (32) erfolgt.

19. Verfahren zur Regelung einer Biogasanlage nach einem der Ansprüche 11 bis 16 mit folgenden Verfahrensschritten:
a) Überwachen des Sauerstoffpartialdrucks in den einzelnen Bioreaktoren (2-i),
b) Absperren des jeweiligen Bioreaktors (2-i) von der Biogasleitung (32), falls der Sauerstoffpartialdruck einen bestimmten Schwellwert übersteigt,
c) Fluten des jeweiligen Bioreaktors (2-I) mit Abgas aus dem Biogasverbraucher (30), und
d) Öffnen des jeweiligen Bioreaktors (2-i).

20. Verfahren zur Regelung einer Biogasanlage nach Anspruch 13 bis 16, mit folgenden Verfahrensschritten:
a) Überwachen von signifikanten Parametern, von aus dem Bioreaktor (2-i) abgeführten Sickersäften,
b) Zumischung von Zusatzstoffen entsprechend der erfassten signifikanten Parameter, und
c) Rückführung des Gemisches in den Bioreaktor (2-i) als Perkolat.

21. Verfahren nach Anspruch 20, **dadurch gekennzeichnet, dass** dem Sickersaft bei Überschreitung eines bestimmten ph-Wertes vor der Rückführung in den Bioreaktor (2-i) Kalziumhydroxid oder Kalkmilch zugesetzt wird.

22. Verfahren nach Anspruch 20 oder 21, **dadurch gekennzeichnet, dass** dem Sickersaft vor Rückführung in den Bioreaktor (2-i) Nährstoffe, insbesondere in Form von Milchzucker, und/oder Methanbildner zugesetzt werden.

## Claims

1. Bioreactor to methanise biomass, with
- a digestion container (2; 200) to accommodate the biomass and which can be sealed in a gas-tight manner by a flap (14);
- a flat-shaped heating device (20) provided in the container wall;
- a bio-gas withdrawal connection (18); and
- a seepage liquor drainage device (22);
**characterized in that**
- the digestion container (2; 200) is constructed from steel-reinforced concrete like a prefabricated garage, and
- **in that** the flat-shaped heating device (20) is integrated in the floor slab (4) of the digestion container (2; 200) in the manner of a floor heating system.

2. Bioreactor according to Claim 1, **characterized in that** the flap (14) is equipped in its edge region with a perimeter sealing tube (130) that can be inflated by means of a fluid, in particular air.

3. Bioreactor according to Claim 2, **characterized in that** the flap (14) has external dimensions that are smaller than the internal dimensions of the digestion container (2; 200) in the plane in which the flap (14) extends in the closed state, so that the container wall (4, 6, 8, 10; 208) projects beyond the flap (14).

4. Bioreactor according to one of the foregoing Claims, **characterized in that** the flap (14) is operable by means of a hydraulic device (16).

5. Bioreactor according to one of the foregoing Claims, **characterized in that** the digestion container (2; 200) has a cubic or rectangular parallelepiped shape with four side-walls (6, 8, 12, 14), a base (4) and ceiling (10; 204) and the flap (14) forms one wall, preferably one vertical side-wall, of the digestion container (2; 200).

6. Bioreactor according to Claim 5, **characterized in that** the ceiling of the digestion container (200) is constructed as a lid (204) that can be raised and lowered by means of lifting cylinders (202), preferably differential cylinders.

7. Bioreactor according to one of the foregoing Claims 1 to 4, **characterized in that** the digestion container is cylinder-shaped and the flap is in the shape of a circular lid covering one outside face of the cylinder.

8. Biogas installation to generate thermal, electrical or mechanical energy from biomass, with
at least one bioreactor (2; 200) to generate biogas from biomass according to one of the foregoing Claims,
a biogas consuming device (30) to generate thermal, electrical or mechanical energy,
a biogas pipe (32) to supply biogas from the at least one bioreactor (2, 200) to the biogas consuming device (30).

9. Biogas installation according to Claim 8, **characterized by**
a valve device (34) in the biogas pipe (32) to regulate the biogas throughput flowrate,
a differential pressure sensor (44) to detect the differential pressure between the pressure prevailing in the at least one bioreactor (2-i) and the ambient pressure, and
a biogas consuming device control equipment (46) to regulate the consumption of biogas in such a way that the differential pressure detected in the differential pressure sensor (44) lies within a defined control interval.

10. Biogas installation according to Claim 9, **characterized in that** the control interval of the differential pressure is positive.

11. Biogas installation according to at least one of the foregoing Claims 8 to 10, with
a partial pressure measuring device (50) to detect the oxygen partial pressure in the at least one bioreactor (2-i),
an exhaust gas purge pipe (54) to supply exhaust gases from the biogas consuming device (34) into the at least one bioreactor (2-i),
a valve equipment (58-i, 60-i) for each bioreactor (2-i), to connect the exhaust gas purge pipe (54) to the respective bioreactor (2-i), and to connect the respective bioreactor (2-i) to the environment, and
a control device (52) to actuate the valve equipment (58-i, 60-i) and to flood the respective bioreactor (2-i) if the oxygen partial pressure in the respective bioreactor (2-i) exceeds a defined value.

12. Biogas installation according to Claim 11, **characterized in that** the valve equipment (58-i, 60-i) for each bioreactor (2-i) comprises a pair of valves consisting of an isolation valve (58-i) in the exhaust gas purge pipe (54) and a purge valve (60-i) to the surrounding environment.

13. Biogas installation according to at least one of the foregoing Claims 8 to 12, with a seepage liquor drainage device (22),
a seepage liquor recycle pipe (28, 74) to recycle the seepage liquor collected by the seepage liquor drainage device (22) into the at least one bioreactor (2-i),
a measuring device (72) to acquire significant parameters of the seepage liquor collected by the seepage liquor drainage device (22), and
an admixing device (70) to feed additives into the seepage liquor recycle pipe (74) in accordance with the parameters acquired by the measuring device (72).

14. Biogas installation according to Claim 13, **characterized in that** the seepage liquor measuring device (72) measures the pH of the seepage liquor and **in that** the admixing device (70) is designed to feed in alkali liquor, in particular calcium hydroxide or milk of lime, if the pH of the seepage liquor falls below a defined value.

15. Biogas installation according to Claim 13 or 14, **characterized in that** the admixing device (70) is designed to feed in nutrients, in particular lactose, for the bacteria that are active in the bioreactors (2-i).

16. Biogas installation according to one of the foregoing Claims 8 to 15, **characterized in that** several biogas consuming devices (30) are provided.

17. Method for controlling a biogas installation according to one of the Claims 8 to 16, with the following process steps:
a) monitoring the differential pressure between the pressure in the interior of the respective bioreactor (2-i) and the surrounding environment, and
b) regulating the amount of biogas consumed by the biogas consuming device (30) in such a way that the differential pressure(s) that is/are monitored remain(s) within a predetermined pressure interval.

18. Method according to Claim 17, **characterized in that** the regulation of the amount of biogas consumed by the biogas consuming device (30) takes place by varying the biogas throughput flowrate in the biogas pipe (32).

19. Method for controlling a biogas installation according to one of the Claims 11 to 16, with the following process steps:
a) monitoring the oxygen partial pressure in the individual bioreactors (2-i),
b) isolating the respective bioreactor (2-i) from the biogas pipe (32) if the oxygen partial pressure exceeds a defined threshold value,
c) flooding the respective bioreactor (2-i) with exhaust gas from the biogas consuming device (30), and
d) opening the respective bioreactor (2-i).

20. Method for controlling a biogas installation according to Claim 13 to 16, with the following process steps:
a) monitoring significant parameters of seepage liquors withdrawn from the bioreactor (2-i),
b) admixing additives in accordance with the significant parameters that are acquired, and
c) recycling the mixture into the bioreactor (2-i) as percolate.

21. Method according to Claim 20, **characterized in that**, if a defined pH is exceeded, calcium hydroxide or milk of lime is added to the seepage liquor before recycling into the bioreactor (2-i).

22. Method according to Claim 20 or 21, **characterized in that** nutrients, especially in the form of lactose and/or methane-forming agents, are added to the seepage liquor before recycling into the bioreactor (2-i).

## Revendications

1. Réacteur biologique pour la biomethanisation de la biomasse, menu de
un récipient putréfié (digesteur) (2 ; 200) verrouillable étanche au gaz par une plaque (14) conçue pour l'admission de la biomasse ;
une installation de chauffage (20) laminaire prévue dans les parois du récipient ;
un raccordement de prélèvement de biogaz (18); et
une installation de drainage de jus de suintement (22); marqué par que le récipient putréfié (digesteur) (2 ; 200) est construit sous forme de garage fini du béton armé, et
que l'installation de chauffage (20) laminaire à façon d'un chauffage par le fond est intégré dans la plaque de base (4) du récipient putréfié (2; 200).

2. d'après l'exigence 1 le réacteur biologique est marqué, par le fait que la plaque (14) dans son secteur marginal est équipée d'un tuyau rendre étanche (130) circulaire gonflable par un fluide, en particulier de l'air,

3. d'après l'exigence 2 le réacteur biologique est marqué, par le fait que la plaque (14) montre des mesures extérieures qui sont plus petites que les mesures intérieur du digesteur (2 ; 200) dans le cas ou la plaque (14) s'élargit pour se fermer, de sorte que la paroi de récipient (4, 6, 8, 10; 208) surmonte la plaque (14).

4. d'après une des exigences précédentes le réacteur biologique marqué, par le fait que la plaque (14) est gérée par une installation d'hydraulique (16).

5. d'après une des exigences précédentes le réacteur biologique marqué, par le fait que le récipient putréfié (2 ; 200) cubique ou quadratique avec quatre parois latérales (6, 8, 12, 14), sol (4) et couverture (10; 204) la plaque (14) forme une paroi, de préférence une paroi latérale verticalement debout du récipient putréfié (2 ; 200) forme.

6. d'après l'exigence 5 le réacteur biologique est marqué, du fait que la couverture un de récipient putréfié (200) est construit comme une couverture (204) qui manoeuvrer (ouverture / fermeture) par des cylindres de soulèvement (202), de préférence cylindres différentiel.

7. d'après une des exigences précédentes 1 à 4 le réacteur biologique est marqué, du fait que le récipient putréfiés montre la forme d'un cylindre et la patte la forme d'une couverture circulaire qui couvre une face du cylindre.

8. Disposition de biogaz visant la production d'une énergie thermique, électrique ou mécanique d'une biomasse, avec au moins un réacteur biologique (2 ; 200) pour la production du biogaz d'une biomasse après une des exigences précédentes. un consommateur de biogaz (30) pour la production d'une énergie thermique, électrique ou mécanique. une conduite de biogaz (32) pour la conduite du biogaz d'au moins d'un réacteur biologique (2, 200) au consommateur de biogaz (30).

9. Installation de biogaz marqué d'après l'exigence 8, par une installation de soupape (34) dans la conduite de biogaz (32) pour régler de débit de biogaz,
une installation de saisie de différence de pression (44) pour la saisie de la différence de pression entre la pression dominante dans au moins un réacteur biologique (2-i) et la pression de l'extérieur, et
un système régulateur de consommateur de biogaz (46) pour le réglage de la consommation du biogaz, de telle manière que la différence de pression saisie par l'installation de saisie de différence de pression (44) se trouve dans un certain intervalle réglementaire.

10. d'après l'exigence 9 l'installation de biogaz est marqué, du fait que l'intervalle réglementaire de la différence de pression est positif.

11. Installation de biogaz est marqué d'après au moins une des exigences 8 précédentes à 10, avec
un système de mesure de pression partielle (50) pour saisir la pression partielle d'oxygène dans au moins un réacteur biologique (2-i),
une conduite de gaz d'échappement de lavage du digesteur (54) pour la conduite des gaz d'échappement du consommateur de biogaz (34) dans au moins un réacteur biologique (2-i),
une installation de soupape (58-i, 60-i) pour chaque réacteur biologique (2-i) pour le raccordement de la conduite de gaz d'échappement de lavage (54) au réacteur biologique respectif (2-i) et pour le raccordement du réacteur biologique respectif (2-i) avec le milieu extérieur,
et à un dispositif de manoeuvres (52) pour actionner l'installation de soupape (58-i, 60-i) et pour inonder le réacteur biologique respectif (2-i), si la pression partielle d'oxygène dans le réacteur biologique respectif (2-i) dépasse une valeur seuil donnée.

12. l'installation de biogaz est marqué d'après l'exigence 11, du fait que l'installation de soupape (58-i, 60-i) pour chaque réacteur biologique (2-i) couvre un couple de soupape composé de la soupape d'arrêt (58-i) dans la conduite de gaz d'échappement (54) et la valve (60-i) à l'extérieur.

13. d'après une des exigences précédentes 8 à 12, Installation de biogaz est marqué avec,
une installation de drainage de jus de suintement (22),
d'une conduite de rapatriement de jus de suintement (28, 74) pour rapatrier le jus de suintement rassemblé par l'installation de drainage de jus de suintement (22) dans au moins un réacteur biologique (2-i),
un système de mesure (72) pour la saisie des paramètres significatifs du jus de suintement, rassemblé par l'installation de drainage de jus de suintement (22), et
d'une installation d'adjonction (70) pour la conduite des additifs dans la conduite de rapatriement de jus de suintement (74) conformément aux paramètres saisis par le système de mesure (72).

14. l'installation de biogaz est marqué d'après l'exigence 13, du fait que le système de mesure de jus de suintement (72) mesure la valeur du PH du jus de suintement, la valeur saisie est livrée à l'installation d'adjonction (70) pour le dosage d'une solution basique, en particulier de l'hydroxyde de calcium ou la chaux cela si la valeur du PH du jus de suintement descend sous une certaine valeur donnée.

15. l'installation de biogaz est marqué d'après l'exigence 13 ou 14, par le fait que l'installation d'adjonction (70) serre à conduire des éléments nutritifs, en particulier du lactose, pour les bactéries actives dans les réacteurs biologiques (2-i).

16. l'installation de biogaz est marqué d'après une des exigences précédentes 8 à 15, que plusieurs consommateurs de biogaz (30) sont prévus.

17. Procédure de règlement d'une installation de biogaz après une les exigences 8 à 16 a les opérations de base suivant :
a) Surveillance de la différence de pression entre la pression à l'intérieur du réacteur biologique respectif (2-i) et à l'extérieur, et
b) réglage de la quantité de biogaz consommé par le consommateur de biogaz (30) de telle manière que la / les différence(s) de pression surveillée(s) restent dans un intervalle de pression prédéterminé.

18. d'après l'exigence 17 le procédé marqué, par le fait que le réglage de la quantité de biogaz consommée par le consommateur de biogaz (30) a lieu par le suivie la variation des débits du biogas dans la conduite de biogaz (32).

19. Procédure de réglage d'une installation de biogaz d'après une des exigences 11 à 16 suivent les opérations de base suivant
a) Surveillance de la pression partielle d'oxygène dans les différents réacteurs biologiques (2-i),
b) isolation du réacteur biologique respectif (2-i) de la conduite de biogaz (32), si dedans la pression partielle d'oxygène dépasse certaine valeur seuil,
c) marées le réacteur biologique respectif (2-i) avec le gaz d'échappement du consommateur de biogaz (30), et
d) ouverture du réacteur biologique respectif (2-i).

20. Procédures de réglage d'une installation de biogaz d'après les exigences 13 à 16, avec les opérations de base suivantes :
a) surveillance des paramètres significatifs, du jus de suintement épuisés du réacteur biologique (2-i)
b) l'adjonction des additifs conformément aux paramètres significatifs saisis, et
c) le rapatriement du mélange dans le réacteur biologique (2-i) comme Perkolat.

21. le procédé est marqué d'après l'exigence 20, du fait que lors d'un dépassement d'une certaine valeur du PH, l'hydroxydes en calcium ou de la chaux sont ajoutés au jus de suintement avant le rapatriement dans le réacteur biologique (2-i)

22. le procédé est marqué d'après l'exigence 20 ou 21, du fait que dans le réacteur biologique (2-i) des éléments nutritifs, sont ajoutés au jus de suintement avant rapatriement, en particulier sous forme de lactose, et/ou producteur de méthane.
